# EUROPEAN PATENT SPECIFICATION

(11) **EP 2 506 013 B1**
(45) Date of publication and mention of the grant of the patent: **31.12.2014**
(21) Application number: 12159450.1
(22) Date of filing: 14.03.2012
(51) Int. Cl.: G01N 33/543, G01N 33/558

(54) **Highly sensitive immunochromatography method**
Hochempfindliches Immunochromatografieverfahren
Procédé immunochromatographique hautement sensible

(30) Priority: 31.03.2011 JP 2011078046
(43) Date of publication of application: 03.10.2012
(73) Proprietor: FUJIFILM Corporation, Tokyo 106-8620 (JP)
(72) Inventor: Wada, Atsuhiko, Kanagawa 258-8577 (JP); Katada, Junichi, Kanagawa 258-8577 (JP)
(74) Representative: Hoffmann Eitle

(56) References cited:
- EP-A1- 0 303 515
- EP-A1- 1 167 970
- EP-B1- 0 733 210
- WO-A1-95/18970
- WO-A1-03/042696
- WO-A1-2008/053900
- WO-A1-2010/001598
- US-A- 3 819 822
- US-A- 5 773 212
- US-A- 5 871 905
- US-B1- 6 284 194
- ARAO SHINSUKE ET AL: "Measurement of urinary lactoferrin as a marker of urinary tract infection", JOURNAL OF CLINICAL MICROBIOLOGY, vol. 37, no. 3, March 1999 (1999-03), pages 553-557, XP002677901, ISSN: 0095-1137

## Description

### BACKGROUND OF THE INVENTION

1. Field of the present invention

The present invention relates to an immunochromatography method that can reduce background density to increase sensitivity.

2. Description of the Related Art

Among immunoassay methods, immunochromatography is generally used in many cases since this method is easily handled and can perform measurement in a short time. As an immune reaction used in immunochromatography, a competitive reaction or a sandwich reaction is widely used. Among these, the sandwich reaction is mainly used in immunochromatography, and as a typical example thereof, the following scheme is operated to detect a substance to be detected formed of an antigen in a sample. First, fine particles sensitized with antibodies to antigens which are substances to be detected are fixed to chromatography carriers as fine solid phase particles, or the antibodies themselves are directly fixed to the chromatography carriers, whereby chromatography carriers having reaction sites are prepared. Meanwhile, fine labeling particles are sensitized with antibodies which can bind specifically to the substances to be detected, whereby sensitized fine labeling particles are prepared. These sensitized fine labeling particles and a sample move together on the chromatography carriers in a chromatographic manner. By these operations, fixed antibodies become a fixed reagent in the reaction sites formed in the chromatography carrier, and the sensitized fine labeling particles bind specifically to the antibodies via antigens which are substances to be detected. Consequently, the presence or degree of signals which are generated when the sensitized fine labeling particles are captured by the reaction sites is visually determined, whereby the presence or amount of substances to be detected included in the sample can be measured.

The above-described immunochromatography has a problem in that high background density is formed due to a gold colloid adsorbed non-specifically onto a non-measurement site, and there is no difference in density between a measurement site and a non-measurement site, which leads to the reduction in the sensitivity for detecting the substance to be measured. In order to solve the problem, there has been a report on a method in which a reagent to which a surfactant has been added moves on the chromatography carrier in a chromatographic manner so as to reduce the background density (JP2005-291783A), but the effect thereof is insufficient.

In the immunochromatography, in order to avoid a problem (risk) that the antigen is not detected due to the low sensitivity, a method of amplifying detection signals is implemented in some cases (JP2002-202307A and Journal of Chromatography, 878 (2010) 271-277). As the signal amplification method, an enzyme such as alkaline phosphatase or peroxidase is used as a label in some cases. In addition, there is a case in which detection is performed by increasing sensitivity by using a compound containing silver or a reductant for silver ions for a label selected from a group consisting of a metallic colloid label and a metallic sulfide label (silver amplification). This type of detection signal amplification method particularly has a problem in that a high background density is formed due to the gold colloid adsorbed non-specifically onto the non-measurement sites, and there is no difference in the density between the measurement sites and the non-measurement sites, which leads to marked reduction in the sensitivity for detecting the substance to be measured.
S. Arao et al., Journal of Clinical Microbiology, Vol. 37, No. 3, March 1999, pages 553-557 describes an immunochromatography test strip device and a method for measuring urinary lactoferrin released from polymorphonuclear leukocytes in a sample diluted with buffer containing 0.07 % CHAPS and 0.07 % BIGCHAP using an antibody which is deposited on an Immunodyne ABC membrane and an antibody-gold conjugate pad.

### SUMMARY OF THE INVENTION

An object of the present invention is to provide an immunochromatography method that enables highly sensitive detection by reducing the background density of non-measurement sites in the immunochromatography method.

The present inventors conducted a thorough investigation to accomplish the above object. As a result, they found that by causing a reaction in the presence of a surfactant having a steroid skeleton, non-specific adsorption of a labeling substance containing a metal onto a non-measurement site can be reduced, and that the background density of the non-measurement sites can be reduced accordingly.

According to the present invention, there is provided an immunochromatography method including developing a complex of a substance to be tested and a labeling substance containing a metal modified with a first binding substance for the substance to be tested on an insoluble carrier in the presence of a surfactant having a steroid skeleton while the substance to be tested and the labeling substance form the complex; and detecting the complex of the substance to be tested by capturing the substance to be tested and the labeling substance in detection sites on the insoluble carrier containing a second binding substance for the substance to be tested or a substance that can bind to the first binding substance for the substance to be tested, and amplifying signals from the captured labeling substance, wherein a solution which contains the substance to be tested and the surfactant and in which the surfactant concentration is 0.1 % by mass to 10% by mass is developed on the insoluble carrier, the labeling substance containing a metal is a metallic colloid and the surfactant is N,N-bis(3-D-gluconamidopropyl)cholamide (BIGCHAP) or 3-[(3-cholamidopropyl)dimethylammonio]propanesulfonate (CHAPS).

Preferably, the insoluble carrier is a porous carrier.

Preferably, the metallic colloid is a gold colloid.

Preferably, the first binding substance is an antibody, and/or the second binding substance is an antibody.

Preferably, the amplifying of the captured labeling substance is performed using an amplification solution that contains a compound containing silver and a reductant for silver ions.

Preferably, the amplification solution is a solution containing a divalent iron ion.

Preferably, during the detection, a labeling substance having an average particle size of 1 µm to 20 µm is detected.

According to the present invention, by reducing the background density of non-measurement sites in the immunochromatography method, the measurement can be performed with a high sensitivity.

### BRIEF DESCRIPTION OF THE DRAWINGS

Fig. 1 shows an assay device used in an example.
Fig. 2 shows an assay device used in an example.

### DESCRIPTION OF THE PREFERRED EMBODIMENTS

Hereinafter, the present invention will be described in more detail.

The immunochromatography method of the present invention is characterized by developing a substance to be tested and a labeling substance containing a metal modified with a first binding substance for the substance to be tested on an insoluble carrier in the presence of a surfactant having a steroid skeleton while the substance to be tested and the labeling substance form a complex.

The surfactant used in the present invention is 3-[(3-cholamidopropyl)dimethylammonio]propanesulfonate (CHAPS) or N,N-bis(3-D-glucoamidopropyl)cholamide (BIGCHAP).

In the present invention a solution containing the substance to be tested and the surfactant can be developed on the insoluble carrier. The surfactant concentration in the solution containing the substance to be tested and the surfactant is not limited as long as the effects of the present invention can be produced. However, the concentration is generally 0.1% by mass to 10% by mass, preferably 0.1% by mass 5.0% by mass, and particularly preferably 0.25% by mass to 1.0% by mass.

### 1. Chromatography

Generally, a chromatography method refers to a technique that simply, rapidly, and specifically determines and measures analytes by the following techniques. That is, a chromatography carrier (insoluble carrier) having at least one reaction site containing a fixed reagent (antibody, antigen, or the like) that can bind to an analyte is used as a stationary phase. On the chromatography carrier, a dispersion in which a labeling substance modified with a reagent that can bind to a substance to be analyzed is dispersed is used as a moving layer so as to move in the chromatography carrier in a chromatographic manner, and the moving layer reaches the reaction sites while the labeling substance binds specifically to the substance to be analyzed: The labeling substance is concentrated in the fixed reagent portion only when the substance to be analyzed exists in a solution to be analyzed since a complex of the substance to be analyzed and the labeling substance binds specifically to the fixed reagent in the reaction sites. The immunochromatography method is a technique that utilizes the above phenomenon and qualitatively and quantitatively analyzes the presence of a substance to be detected included in a solution to be analyzed by using visual observation or an appropriate instrument.

The kit or device for implementing the immunochromatography method of the present invention may include a compound containing silver or a reductant for silver ions. In the kit or device, signals are amplified by an amplification reaction using, as a nucleus, the complex of the substance to be analyzed and the labeling substance binding to the fixed reagent, and as a result, high sensitivity can be accomplished. According to the present invention, highly sensitive immunochromatography can be performed rapidly.

2. Sample to be tested

The sample to be tested that can be analyzed by the immunochromatography method of the present invention is not particularly limited as long as the sample is likely to include a substance to be analyzed. Examples of the sample include biochemical samples, particularly, body fluid of animals (particularly, humans) (for example, blood, serum, plasma, cerebrospinal fluid, tears, sweat, urine, pus, nasal secretions, or phlegm), excrement (for example, stools), organs, tissues, mucous membranes or skin, scraped specimens (swabs) considered to include the above samples, gargling liquid, animals or plants themselves, or dried substances thereof.

3. Post-treatment of sample to be tested

In the immunochromatography method of the present invention, the sample to be tested can be used as it is or used in the form of an extraction solution that is obtained by extracting the sample to be tested by using an appropriate solvent for extraction, in the form of a diluted solution obtained by diluting the extraction solution with an appropriate diluent, or in the form of a concentrate obtained by concentrating the extraction solution by an appropriate method. As the solvent for extraction, solvents used in general immunological assays (for example, water, physiological saline, a buffer solution, and the like) or water-miscible organic solvents that can directly cause an antigen-antibody reaction by being diluted with the above solvents can be used.

4. Constitution

A chromatography strip that can be used in the immunochromatography method of the present invention is not particularly limited as long as the chromatography strip is usable for general chromatography. For example, the chromatography strip can be disposed on an adhesive sheet in an order of a sample-adding pad, a labeled substance-holding pad (for example, a gold colloid antibody-holding pad), a chromatography carrier (for example, antibody-fixed membrane), and an absorption pad, from the upstream toward the downstream in the developing direction. The chromatography carrier includes a capturing site, a detection zone (also described as a detection portion in some cases) as an area where an antibody or an antigen binding specifically to the substance to be analyzed is fixed. If desired, the carrier further includes a control zone (also described as a control portion in some cases) as an area where a control antibody or antigen is fixed. The labeled substance-holding pad can be prepared by preparing a suspension containing a labeled substance, applying the suspension to an appropriate absorption pad (for example, a glass fiber pad), and then drying the resultant. As the sample-adding pad, for example, a glass fiber pad can be used.

### 4-1. Labeling substance

As the labeling substance used in the present invention, a metallic colloid label is used as a label used for labeling a substance to be detected that binds specifically to a substance to be tested (antigen). The metallic colloid label is not particularly limited as long as the label can be used in general chromatography. Examples of the metallic colloid label include a platinum colloid, a gold colloid, a silver colloid, an iron colloid, an aluminum hydroxide colloid, or the like. In the immunochromatography of the present invention, one or more of the metallic colloid label can be used as the label. The metallic colloid can be caused to bind to a specific binding substance according to a method known in the related art (for example, The Journal of Histochemistry and Cytochemistry, Vol. 30, No. 7, pp 691-696, (1982)).

### 4-2. Binding substance

In the present invention, the labeling substance is modified with the first binding substance for the substance to be tested. Any compound can be used as the first binding substance as long as the compound exhibits affinity with the substance to be tested, such as an antibody to the substance to be tested (antigen), an antigen to the substance to be tested (antibody), an aptamer to the substance to be tested (a protein, a low-molecular weight compound, or the like), and the like.

In the present invention, the insoluble carrier includes (a) the second binding substance for the substance to be tested or (b) a substance that can bind to the first binding substance. Any compound can be used as the second binding substance for the substance to be tested as long as the compound exhibits affinity with the substance to be tested, such as an antibody to the substance to be tested (antigen), an antigen to the substance to be tested (antibody), an aptamer to the substance to be tested (a protein, a low-molecular weight compound, or the like), and the like. Moreover, the second and first binding substances may be different from or the same as each other. The substance that can bind to the first binding substance for the substance to be tested may be the substance to be tested itself or a compound having a site that the first binding substance recognizes. Examples of such a substance include a compound obtained by combining a derivative of the substance to be tested with a protein (for example, BSA), and the like.

Preferably, the first binding substance is an antibody, and/or the second binding substance is an antibody. In the immunochromatography method of the present invention, the antibody having specificity against the substance to be analyzed is not particularly limited. For example, an antiserum prepared from the serum of an animal immunized with the substance to be analyzed, an immunoglobulin fraction purified from an antiserum, a monoclonal antibody that is obtained by cell fusion using splenocytes from an animal immunized with the substance to be analyzed, or a fragment thereof (for example, F(ab')2, Fab, Fab', or Fv) can be used. These antibodies can be prepared by a common method.

4-3. Chromatography carrier

As the chromatography carrier, insoluble carriers are used, and among these, a porous carrier is preferable. Particularly, a nitrocellulose membrane, a cellulose membrane, an acetylcellulose membrane, a polysulfone membrane, a polyether sulfone membrane, a nylon membrane, a glass fiber, an unwoven fabric, cloth, or a yarn is preferable.

Generally, a substance for detection is fixed to a portion of the chromatography carrier, thereby forming a detection zone. The substance for detection may be directly fixed to a portion of the chromatography carrier physically or via a chemical bond, Alternatively, the substance for detection may be physically or chemically bonded to fine particles such as latex particles, and then these fine particles may be trapped in a portion of the chromatography carrier so as to be fixed. In addition, it is preferable to use a chromatography carrier that has undergone a treatment for preventing non-specific adsorption by being treated with an inert protein after the fixation of the substance for detection.

4-4. Sample-adding pad

Examples of materials of the sample-adding pad include those have uniformity such as cellulose filter paper, glass fiber, polyurethane, polyacetate, cellulose acetate, nylon, and cotton cloth, but the materials are not limited to these. The sample-adding portion not only takes a sample added thereto that includes the substance to be analyzed but also has a function of filtering particles of insoluble substances in the sample. In addition, in order to prevent decrease in the accuracy of detection that is caused when the substance to be analyzed in the sample is non-specifically adsorbed onto the material of the sample-adding portion during the analysis, the material constituting the sample-adding portion is used after being treated in advance to prevent the non-specific adsorption in some cases.

4-5. Labeled substance-holding pad

Examples of materials of the labeled substance-holding pad include cellulose filter paper, glass fiber, unwoven fabric, and the like. The material is impregnated with a certain amount of the labeling substance prepared as described above, followed by drying, thereby preparing the labeled substance-holding pad.

4-6. Absorption pad

The absorption pad is a site that physically absorbs the sample added thereto by chromatographic movement and absorbs and removes an unreacted labeling substance or the like that is not insolubilized in the detection portion of the chromatography carrier. For the absorption pad, water-absorbing materials such as cellulose filter paper, unwoven fabric, cloth, and cellulose acetate are used. The chromatographic speed from when the chromatographic leading end of the added sample reaches the absorption portion varies with the material, size, or the like of the absorber. Accordingly, the speed suitable for the measurement of the substance to be analyzed can be set by appropriately selecting the material and size.

5. Method of immunological test

Hereinafter, as a specific embodiment of the immunochromatography method of the present invention, a sandwich method will be described.

Though not particularly limited, the substance to be tested can be analyzed by the following order in the sandwich method. First, first and second antibodies having specificity against the substance to be tested (antigen) are prepared in advance by the method described above, and the first antibody is labeled in advance. The second antibody is fixed onto an appropriate first insoluble carrier (for example, a nitrocellulose membrane, a glass fiber membrane, a nylon membrane, a cellulose membrane, or the like), and brought into contact with a sample to be tested (or an extraction solution thereof) that is likely to include a substance to be tested (antigen). In this case, if the substance to be tested exists in the sample to be tested, an antigen-antibody reaction occurs. This antigen-antibody reaction can be caused in the same manner as in a general antigen-antibody reaction. An excess amount of the labeled first antibody is brought into contact with the sample to be tested simultaneously with or after the antigen-antibody reaction, and in this case, if the substance to be tested exists in the sample to be tested, an immunocomplex including the fixed second antibody, the substance to be tested (antigen), and the labeled first antibody is formed.

In the sandwich method, after the reaction between the fixed second antibody, the substance to be tested (antigen), and the first antibody is completed, the labeled first antibody not forming the immunocomplex is removed, and then a first optical density measurement is performed on the area of the first insoluble carrier where the fixed second antibody is fixed. In this manner, the labeling substance can be quantitated, and the amount of the substance to be tested included in the sample to be tested can be measured. Thereafter, by supplying metal ions and a reductant, signals from the label of the labeled first antibody forming the immunocomplex are amplified, and then a second optical density measurement is performed. In this manner, the labeling substance having undergone the amplification can be quantitated, and the amount of the substance to be tested included in the sample to be tested can be measured.

6. Washing

In the present invention, after the developing of the substance to be tested and the labeling substance containing a metal modified with a first binding substance for the substance to be tested on an insoluble carrier in the presence of a surfactant having a steroid skeleton while the substance to be tested and the labeling substance form a complex, a washing solution may be developed on the insoluble carrier, followed by the amplification of the captured labeling substance.

(Washing Solution)

Any solution may be used as the washing solution for removing the labeled antibody not forming the immunocomplex as long as the solution has a function of washing.

The washing solution is not particularly limited so long as the solution is a liquid for washing the labeling substance that remains in the chromatography carrier by a route other than the specific binding reaction, that is, the labeling substance that remains nonspecifically. The washing solution may simply be a single solvent such as water or ethanol, and for example, a solution such as a PBS buffer containing 1% BSA or a surfactant can be used. Moreover, as the washing solution, a liquid containing silver ions or a liquid containing a reductant of silver ions, which will be described later, can also be used. The washing solution is developed while washing the labeling substance that remains nonspecifically during the development, so the washing solution is developed in a state of containing the labeling substance. However, as the washing solution that has not been developed, a liquid not containing the labeling substance is used so as to enhance the washing effect. In addition, in order to enhance the washing effect, a washing solution that has been adjusted in terms of pH, or a washing solution to which a surfactant component, a protein such as BSA, or a high-molecular weight compound such as polyethylene glycol has been added may be used.

(Development of Washing Solution, and the Direction)

The washing solution is added to a chromatography strip after the development of the specimen solution so as to wash out the labeling substance remaining in the chromatography strip, which is a labeling substance other than the substances combined by the antigen-antibody reaction. As the method of sending the washing solution, there are a method of adding the washing solution to a sample-dripping portion in a state where the specimen solution has been developed; a method of attaching in advance a washing solution-adding pad and a water-absorbing pad for sending the washing solution to the strip, adding the washing solution to the washing solution-adding pad, and sending the washing solution toward the water-absorbing pad; a method of providing in advance a washing solution-adding site in the strip and adding the washing solution in the washing solution-adding site after the specimen solution is developed; and the like. A more preferable method is a method of attaching the washing solution-adding pad and the water-absorbing pad for sending the washing solution to the strip after the specimen solution is developed on the strip, supplying the washing solution to the washing solution-adding pad, and developing the washing solution. As the method of supplying the washing solution to the washing solution-adding pad, the washing solution-adding pad may be inserted in a pot containing the washing solution, or the washing solution may be dripped to the washing solution-adding pad.

In the present specification, the development direction of the solution of the substance to be tested is defined as a direction connecting the sample-adding pad with the absorption pad, and the development direction of the washing solution is defined as a direction connecting the washing solution-adding pad with the water-absorbing pad for sending the washing solution.

When the angle formed between the development direction of the solution of the substance to be tested and the development direction of the washing solution is from 45° to 170°C, the washing effect is increased. In addition, the angle formed between the development direction of the solution of the substance to be tested and the development direction of the washing solution is preferably from 60°C to 170°, and more preferably 60° to 150°.

Any material can be used as the washing solution-adding pad (also described as the second insoluble carrier) as long as the washing solution can be added to the material. A glass fiber pad, a cellulose membrane, a nitrocellulose membrane, or the like can be used as the washing solution-adding pad.

Any material can be used as the water-absorbing pad as long as the material can absorb water. Cellulose, nitrocellulose, glass fiber, or a combination thereof can be used as the water-absorbing pad.

7. Amplification solution

The chemicals contained in the amplification solution react catalytically due to the action of the labeling substance or the substance to be tested. In this manner, the amplification solution can amplify signals by generating colored compounds or emitting light. Examples of the amplification solution include a silver ion solution that causes the precipitation of metallic silver on a metallic label by a physical phenomenon, a solution of a phenylenediamine compound and a naphthol compound, which turns into a pigment due to the action of a peroxidase label and peroxide, and the like.

Specifically, a so-called photographic-developer disclosed in general publications in the field of photographic chemistry (for example, "Revised Fundamentals of Photographic Engineering-Silver Halide Photograph" (The Society of Photographic Science and Technology of Japan, Corona Publishing Co., Ltd.), "Photographic Chemistry" (Sasai Akira, Photographic Industry Publishing Company), and "The Latest Prescription Handbook" (Kikuchi Shinichi et al, Amiko Publishing Company) can be used as the amplification solution. Any photographic-developer can be used as the amplification solution without particular limitation as long as the photographic-developer is a so-called physical-developer including silver ions that are reduced mainly to a metallic colloid which acts as a nucleus for development.

As a specific example of the amplification solution, an amplification solution that includes a compound containing silver and a reductant for silver ions can be used. The compound containing silver and the reductant for silver ions will be described below.

(Compound Containing Silver (Ion))

As the compound containing silver ions, an organic silver salt, an inorganic silver salt, or a silver complex can be used. A silver ion-containing compound showing high solubility against a solvent such as water is preferable, and examples thereof include silver nitrate, silver acetate, silver lactate, silver butyrate, silver thiosulfate, and the like, and among these, silver nitrate is particularly preferable. As the silver complex, a silver complex coordinated to a ligand having a water-soluble group such as a hydroxyl group or a sulfone group is preferable, and examples thereof include hydroxyl thioether silver and the like.

The inorganic silver salt or the silver complex is contained as silver generally at 0.001 mol/m² to 0.2 mol/m², and preferably at 0.01 mol/m² to 0.05 mol/m².

(Reductant for Silver Ions)

As the reductant for silver ions, any of inorganic and organic materials, or a mixture thereof can be used so long as the material can reduce silver ions to silver.

Preferable examples of the inorganic reductant include a reductive metal salt and a reductive metal complex salt that can change atomic valence by using a metal ion, such as Fe²⁺, V²⁺, and Ti³⁺. When the inorganic reductant is used, it is necessary to remove or detoxify oxidized ions by forming a complex or reducing the ions. For example, in a system using Fe²⁺ as a reductant, ions can be detoxified by forming a complex of Fe³⁺ which is an oxide by using citric acid or EDTA. In the present system, it is preferable to use this type of inorganic reductant, and it is more preferable to use a metal salt of Fe²⁺.

In addition, developing agents used for wet silver halide photosensitive materials (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials clearly known to a person skilled in the art, for example, materials disclosed in US Patent No. 6,020,117, can also be used.

As the reductant, an ascorbic acid reductant is also preferable. Useful ascorbic acid reductants includes ascorbic acid, ascorbic acid-like substances and isomers and derivatives thereof. Preferable examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related art), an enediol type of ascorbic acid, an enaminol type of ascorbic acid, a thioenol type of ascorbic acid, and the like. Particularly, D-, L-, or D,L-ascorbic acid (and an alkali metal salt thereof) or isoascorbic acid (or an alkali metal salt thereof) is preferable, and a sodium salt thereof is a preferable salt. If necessary, a mixture of these reductants can be used.

8. Other auxiliary agents

Other auxiliary agents of the amplification solution includes a buffer or a preservative, for example, an antioxidant, an organic stabilizer, or a speed adjuster. As the buffer, for example, acetic acid, citric acid, sodium hydroxide or a salt of any of these, a buffer using tris(hydroxyrnethyl)aminomethane, or other buffers used for general chemical experiments can be used. The amplification solution can be adjusted to an optimal pH by appropriately using these buffers. In addition, as an anti-fogging agent, an alkylamine can be used as an additive, and a particularly preferable anti-fogging agent is dodecylamine. In order to improve the solubility of these additives, a surfactant can be used, and a particularly preferable surfactant is C₉H₁₉-C₆H₄-O-(CH₂CH₂O)₅₀H.

9. Method of calculating average particle size during detection

During the detection (after amplification), a test line portion is cut off, and the rear surface of a sample is adhered to a stage by a carbon paste. Subsequently, a section of the sample is cut, followed by carbon deposition, and the shape and size thereof is observed by a scanning electron microscope. For example, by using an FE-STEM S-5500 manufactured by Hitachi High-Technologies Corporation., the SEM observation of the sample surface can be performed at an accelerating voltage of 10 KV using reflection electrons. Thereafter, 100 signal particles are selected to measure a circle equivalent diameter of the projected area of the particles, and the average thereof is calculated, whereby the calculated value is taken as an average particle size at the time of detection.

The present invention will be more specifically described based on the following examples, but the present invention is not limited to the examples.

### [Examples]

(1) Preparation of immunochromatography kit detecting influenza virus antigen

(1-1) Preparation of gold colloid modified with anti-influenza A antibody

(1-1-1) Preparation of F(ab')₂ fragment anti-influenza A virus antibody

An F(ab')₂ fragment anti-influenza A virus antibody was prepared using an anti-influenza A virus antibody (product number 7307, manufactured by Medix Biochemica) and an ImmunoPure IgG1 Fab and F(ab')₂ Preparation Kit (product number 44880, Pierce Biotechnology, Inc.).

(1-1-2) Preparation of gold colloid modified with anti-influenza A antibody fragment

A 160 µg/mL F(ab')₂ fragment anti-influenza A virus antibody solution prepared in (1-1-1) was added at 1 mL to a gold colloid solution of which the pH was adjusted by adding 1 mL of a 50 mM KH2PO4 buffer (pH 7.5) to 9 mL of a solution of a gold colloid having a diameter of 50 nm (EM. GC50, British Biocell International), followed by stirring. The solution was allowed to stand for 10 minutes, and then 550 µL of a 1% polyethylene glycol (PEG Mw. 20000, product number 168-11285, Wako Pure Chemical Industries, Ltd.) aqueous solution was added thereto, followed by stirring. Thereafter, 1.1 mL of a 10% bovine serum albumin (BSA Fraction V, product number A-7906, SIGMA) aqueous solution was added thereto, followed by stirring. This solution was subjected to centrifugation by a centrifuge (Himac CF16RX, Hitachi) at a centrifugal force of 8000 times gravity, 4°C for 30 minutes, the supernatant was then removed excluding approximately 1 mL thereof, and the gold colloid was dispersed again by an ultrasonic washing machine. Subsequently, the gold colloid was dispersed in 20 mL of a gold colloid storage solution (20 mM Tris-HCl buffer (pH 8.2), 0.05% PEG (Mw. 20000), 150 mM NaCl, 1% BSA) and subjected to centrifugation again at a centrifugal force of 8000 times gravity, 4°C for 30 minutes. Then the supernatant was removed excluding approximately 1 mL thereof, and the gold colloid was dispersed again by an ultrasonic washing machine, thereby obtaining an antibody-modified gold colloid (50 nm) solution.

(1-2) Preparation of gold colloid modified with anti-H5 influenza antibody

(1-2-1) Preparation of F(ab')₂ fragment anti-H5 influenza virus antibody

An F(ab')₂ fragment anti-H5 influenza virus antibody was prepared using an anti-H5 influenza virus antibody (product number AM00942PU-N, Acris Antibodies) and Pierce F(ab')₂ Preparation Kit (product number 44988, Thermo Scientific)

(1-2-2) Preparation of gold colloid modified with anti-H5 influenza virus antibody

A 4 µg/mL F(ab')₂ fragment anti-H5 influenza virus antibody solution prepared in (1-2-1) was added at 1 mL to a gold colloid solution of which the pH was adjusted by adding 1 mL of a 20 mM borax buffer (pH 9.0) to 9 mL of a solution of gold colloid having a diameter of 50 nm (EM. GC50, British Biocell International), followed by stirring. The solution was allowed to stand still for 10 minutes, and then 550 µL of a 1% polyethylene glycol (PEG Mw. 20000, product number 168-11285, Wako Pure Chemical Industries, Ltd.) aqueous solution was added thereto, followed by stirring. Thereafter, 1.1 mL of a 10% bovine serum albumin (BSA Fraction V, product number A-7906, SIGMA) aqueous solution was added thereto, followed by stirring. This solution was subjected to centrifugation by a centrifuge (Himac CF16RX, Hitachi) at a centrifugal force of 8000 times gravity, 4°C for 30 minutes, the supernatant was then removed excluding approximately 1 mL thereof, and the gold colloid was dispersed again by an ultrasonic washing machine. Subsequently, the gold colloid was dispersed in 20 mL of a gold colloid storage solution (20 mM Tris-HCl buffer (pH 8.2), 0.05% PEG (Mw. 20000), 150 mM. NaCl, 1% BSA) and subjected to centrifugation again at a centrifugal force of 8000 times gravity, 4°C for 30 minutes. Then the supernatant was removed excluding approximately 1 mL thereof, and the gold colloid was dispersed again by an ultrasonic washing machine, thereby obtaining an antibody-modified gold colloid (50 nm) solution.

(1-3) Preparation of pad holding gold colloid modified with anti-influenza A antibody fragment

The gold colloid modified with an anti-influenza A antibody, which was prepared in (1-1), was diluted with a gold colloid application solution (20 mM Tris-HCl buffer (pH 8.2), 0.05% PEG (Mw. 20000), 5% sucrose) and water so as to yield an OD at 520 nm of 6.0. This solution was evenly applied by 0.8 mL to each sheet of glass fiber pad (Glass Fiber Conjugate Pad, Millipore Corporation) cut to 8 mm×150 mm, and the resultant was dried overnight under reduced pressure, thereby obtaining a gold colloid antibody-holding pad.

(1-4) Preparation of pad holding gold colloid modified with anti-H5 influenza virus antibody

The gold colloid modified with an anti-H5 influenza antibody, which was prepared in (1-2), was diluted with a gold colloid application solution (20 mM Tris-HCl buffer (pH 8.2), 0.05% PEG (Mw. 20000), 5% sucrose) and water so as to yield an OD at 520 nm of 6.0. This solution was evenly applied by 0.8 mL to each sheet of glass fiber pad (Glass Fiber Conjugate Pad, Millipore Corporation) cut to 8 mm×150 mm, and the resultant was dried overnight under reduced pressure, thereby obtaining a gold colloid antibody-holding pad.

(1-5) Preparation of antibody-fixed membrane (chromatography carrier)

(1-5-1) Preparation of anti-influenza A antibody-fixed membrane

The antibody was fixed to a nitrocellulose membrane (including a plastic lining, HiFlow Plus HF120, Millipore Corporation) cut to 25 mm×200 mm by the following method, thereby preparing an antibody-fixed membrane. The long side of the membrane became the bottom, and at a position 7 mm above the bottom, an anti-influenza A monoclonal antibody (Anti-Influenza A SPTN-5 7307, Medix Biochemica) solution to be fixed that was prepared at 1.5 mg/mL was applied in a shape of a line having a width of approximately 0.7 mm by using an ink jet type applicator (BioDot). Likewise, at a position 13 mm above the bottom, an anti-mouse IgG antibody (anti-mouse IgG(H+L), rabbit F(ab')2, product number 566-70621, Wako Pure Chemical Industries, Ltd.) solution for control that was prepared at 0.5 mg/mL was applied in a line shape. The membrane applied with these solutions was dried by a hot air drier at 50°C for 30 minutes. A blocking solution (50 mM boric acid buffer (pH 8.5) containing 0.5 w% casein (derived from milk, product number 030-01505, Wako Pure Chemical Industries, Ltd.)) was put in the pad at 500 mL and then allowed to stand as it was for 30 minutes. Subsequently, the membrane was moved so as to be dipped in 500 mL of a washing and stabilizing solution (50 mM Tris-HCl (pH 7.5) buffer containing 0.5 w% sucrose and 0.05 w% sodium cholate) that was put in the same pad and then allowed to stand as it was for 30 minutes. The membrane was then taken out of the solution, and then dried overnight at room temperature, thereby obtaining an antibody-fixed membrane.

(1-5-2) Preparation of anti-H5 influenza virus antibody-fixed membrane (chromatography carrier)

The antibody was fixed to a nitrocellulose membrane (including a plastic lining, HiFlow Plus HF120, Millipore Corporation) cut to 25 mm×200 mm by the following method, thereby preparing an antibody-fixed membrane. The long side of the membrane became the bottom, and at a position 7 mm above the bottom, an anti-influenza A monoclonal antibody (Mouse monoclonal [15A6] to Influenza A virus Hemagglutinin, Abcam plc.) solution to be fixed that was prepared at 1.5 mg/mL was applied in a shape of a line having a width of approximately 0.7 mm by using an ink jet type applicator (BioDot). Likewise, at a position 13 mm above the bottom, an anti-mouse IgG antibody (anti-mouse IgG(H+L), rabbit F(ab')2, product number 566-70621, Wako Pure Chemical Industries, Ltd.) solution for control that was prepared at 0.5 mg/mL was applied in a line shape. The membrane applied with these solutions was dried by a hot air drier at 50°C for 30 minutes. A blocking solution (50 mM boric acid buffer (pH 8.5) containing 0.5 w% casein (derived from milk, product number 030-01505. Wako Pure Chemical Industries, Ltd.)) was put in the pad at 500 mL and then allowed to stand as it was for 30 minutes. Subsequently, the membrane was moved so as to be dipped in 500 mL of a washing and stabilizing solution (50 mM Tris-HCl (pH 7.5) buffer containing 0.5 w% sucrose and 0.05 w% sodium cholate) that was put in the same pad and then allowed to stand as it was for 30 minutes. The membrane was then taken out of the solution, and then dried overnight at room temperature, thereby obtaining an antibody-fixed membrane.

(1-6) Preparation of immunochromatography strip

The antibody-fixed membrane prepared in (1-5) was attached to a back adhesive sheet (ARcare 9020, NIPPN/TechnoCluster, Inc.). At this time, in the long side of the membrane, a test line side became the bottom. The gold colloid antibody-holding pads prepared in (1-3) and (1-4) were attached to the bottom of the antibody-fixed membrane such that the thickness thereof became approximately 2 mm, and a sample-adding pad (glass fiber pad (Glass Fiber Conjugate Pad, Millipore Corporation) cut to 18 mm×250 mm) was attached over the bottom of the gold colloid antibody-holding pad such that the thickness thereof became approximately 4 mm. Moreover, an absorption pad (a cellulose-glass membrane (CF6, Whatman plc) cut to 80 mm×250 mm) was attached over the top of the antibody-fixed membrane such that the thickness thereof became approximately about 5 mm.

These members (immunochromatography main body members) attached to each other in layers were cut with a guillotine type cutter (CM4000, NIPPN/TechnoCluster, Inc.) parallel to the short side such that the long side of the members had a width of 25 mm, thereby preparing immunochromatography strips with a size of 25 mm×55 mm. These strips were taken as an anti-influenza A immunochromatography kit and an anti-H5 influenza immunochromatography kit respectively.

(1-7) Preparation of silver amplification solution

(1-7-1) Preparation of reductant solution

In 290 g of water, 23.6 ml of a 1 mol/l aqueous iron nitrate solution prepared by dissolving iron (III) nitrate nonahydrate (Wako Pure Chemical Industries, Ltd., 095-00995) in water and 13.1 g of citric acid (Wako Pure Chemical Industries, Ltd., 038-06925) were dissolved. After the above components were completely dissolved, 36 ml of nitric acid (10% by mass) was added thereto under stirring using a stirrer, and 60.8 g of ammonium iron (II) sulfate hexahydrate (Wako Pure Chemical Industries, Ltd., 091-00855) was further added thereto, thereby obtaining a reductant solution.

(1-7-2) Preparation of silver ion solution

To 66 g of water, 8 ml of a silver nitrate solution (containing 10 g of silver nitrate) and 24 ml of a 1 mol/l aqueous iron nitrate solution were added. This solution was mixed with a solution that had already been prepared by dissolving 5.9 ml of nitric acid (10% by mass), 0.1 g of dodecylamine (Wako Pure Chemical Industries, Ltd., 123-00246), and 0.1 g of a surfactant C₁₂H₂₅-C₆H₄-O-(CH₂CH₂O)₅₀H in 47.6 g of water, thereby preparing a silver ion solution.

(1-8) Preparation of assay device

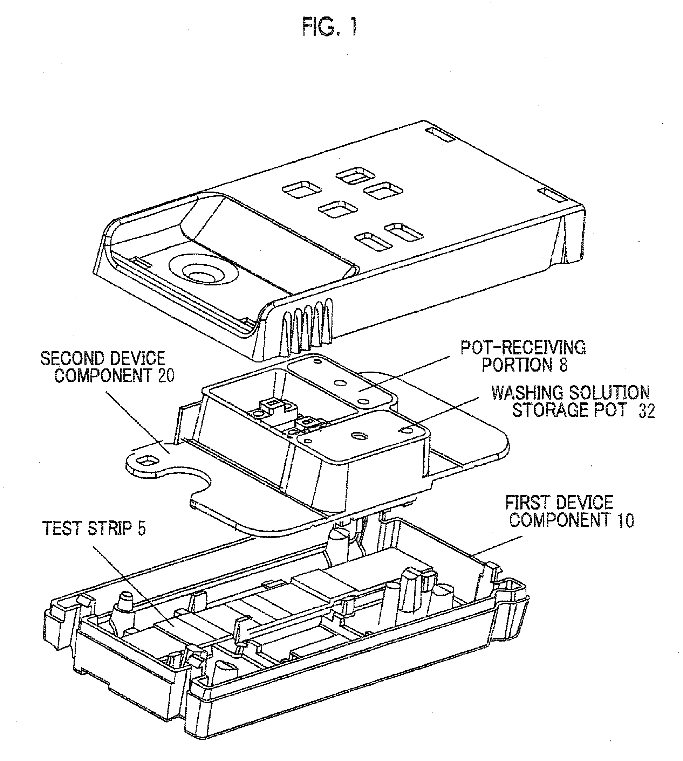

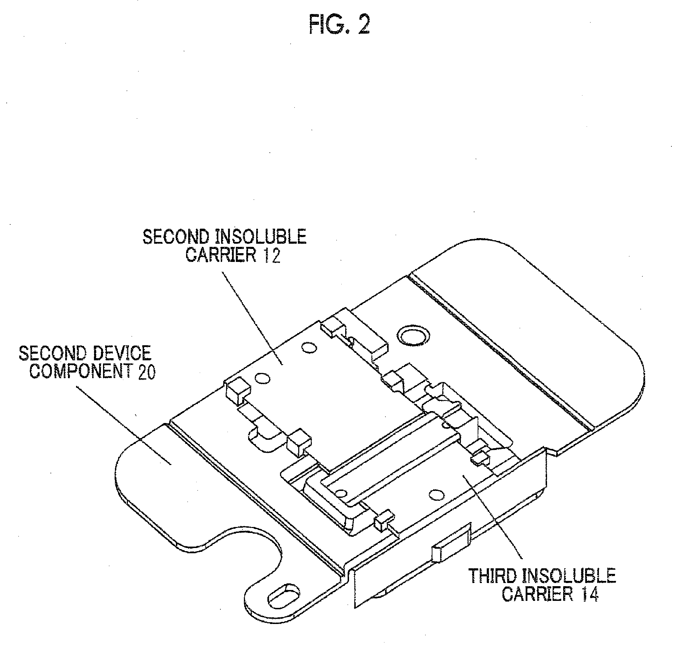

As shown in Fig. 1, the immunochromatography test strip 5 was loaded in the first device component (made of polypropylene through injection molding) shown in Fig. 1. Thereafter, as shown in Fig 2, glass fiber pads (Glass Fiber Conjugate Pad, Millipore Corporation) were loaded as the second and third insoluble carriers 14. To the washing solution storage pot of Fig. 1, 185 µL of the reductant solution prepared in the above (1-7-1) was introduced. The pot was sealed by means of heat lamination by using an aluminum laminate sheet (NewADM, Sunrise Company), and the second device component was fit into the first device component. In addition, to the pot-receiving portion 8 shown in Fig. 1, 95 µL of the silver ion solution prepared in the above (1-7-2) was introduced, and a liquid storage pot sealed with an aluminum laminate sheet was loaded in the portion.

### (2) Evaluation

### (2-1) Dropping

A mock-positive specimen (BD Flu examen control A+B- (Becton, Dickinson and Company)) or an H5 influenza HA protein was diluted with an extraction solution in the manner described below. In the sample-adding pad of the immunochromatography kit for test prepared in (1-6), 140 µL of the solutions diluted with the respective extraction solutions were dropped in a specimen-injecting hole and allowed to stand for 10 minutes. Specifically, the following Comparative Examples 1 and 2, and Examples 1, 2, and 3 were performed.

### (Comparative Example 1)

As an extraction solution, a liquid that was obtained by adding SDS (surfactant not having a steroid skeleton) to a PBS buffer containing 1% BSA such that the proportion of SDS became 1% was used. The antigen (mock-positive specimen (BD Flu examen control A+B-(Becton, Dickinson and Company))) was diluted with the extraction solution and dropped onto the anti-influenza A immunochromatography kit as described above.

### (Comparative Example 2)

A PBS buffer containing 1% BSA was used as an extraction solution. The antigen (H5 influenza HA protein) was diluted with the extraction solution and dropped onto the anti-H5 influenza immunochromatography kit as described above.

### (Reference Example 1)

As an extraction solution, a liquid that was obtained by adding deoxycholic acid (surfactant having a steroid skeleton) to a PBS buffer containing 1% BSA such that the proportion of deoxycholic acid became 1% was used. The antigen (mock-positive specimen (BD Flu examen control A+B- (Becton, Dickinson and Company))) was diluted with the extraction solution and dropped onto the anti-influenza A immunochromatography kit as described above.

### (Example 2)

As an extraction solution, a liquid that was obtained by adding CHAPS (surfactant having a steroid skeleton) to a PBS buffer containing 1% BSA such that the proportion of CHAPS became 0.5% was used. The antigen (H5 influenza HA protein) was diluted with the extraction solution and dropped onto the anti-H5 influenza immunochromatography kit as described above.

(Example 3)

As an extraction solution, a liquid that was obtained by adding BIGCHAPS (surfactant having a steroid skeleton) to a PBS buffer containing 1% BSA such that the proportion of BIGCHAPS became 0.5% was used. The antigen (H5 influenza HA protein) was diluted with the extraction solution and dropped onto the anti-H5 influenza immunochromatography kit as described above.

(2-2) Pressing second and third insoluble carriers

The specimen solution was developed for 10 minutes, and thereafter, a pressing portion 34 of a second device component 20 was pressed with a force of 10 N so as to bend the second device component 20, thereby pressing the insoluble carrier for sending a solution (a second insoluble carrier 12) on the membrane. At this time, the degree of pressing was restricted by the contact between a rib 15 of a first device component 10 and the second device component 20. The height of the rib member 15 was adjusted such that the membrane surface of the immunochromatography strip (a first insoluble carrier 11) and a portion that contacts the membrane of the insoluble carrier for sending a solution due to the second device component was crushed to 0.2 mm. Moreover, the shape of a projection portion of the second device component was adjusted such that a clearance between the membrane surface of the immunochromatography strip (the first insoluble carrier 11) and a pressing surface 18a of the second device component became 0.005 mm.

(2-3) Washing

A seal breaking portion 19 of the second device component 20 was pressed after 30 seconds elapsed from when the insoluble carrier for sending a solution was pressed, whereby the insoluble carrier for sending a solution and the aluminum sheet were broken, and the second insoluble carrier 12 was dipped in the reductant solution. In this manner, the reductant solution was sent for 3 minutes by being developed on the immunochromatography strip. By this process, the immunochromatography strip was dipped in the reductant solution, and materials that had not been specifically adsorbed were washed away.

(2-4) Signal amplification caused by amplification solution

A solution storage pot 32 loaded in the second device component 20 was pushed in, and the seal thereof was broken due to the projection portion, whereby the silver ion solution was discharged from the solution storage pot and injected into the injection hole. The silver ion solution was filled in the clearance between the membrane surface and the projection portion of the second device component, and the gold colloid label adsorbed onto the detection line was amplified for 1 minute.

### (2-5) Evaluation of background density and test line density

The membrane portion of the immunochromatography strip having undergone the amplification process was cut and sufficiently washed with water. The anti-influenza A antibody-fixed membrane that had been washed with water was placed on a black plate, and an image thereof was captured using a LAS-4000 (FUJIFILM Corporation). In addition, the anti-H5 influenza antibody-fixed membrane that had been washed with water was placed on a white plate, and an image thereof was captured using a LAS-4000 (FUJIFILM Corporation). The background density (OD) of the respective membranes was measured.

In addition, the value of the background density was subtracted from the value measured from the test line, thereby calculating a test line density (ΔOD). In a case of the anti-influenza A immunochromatography kit, the test line density (ΔOD) is the density obtained when the mock-positive specimen (BD Flu examen control A+B- (Becton, Dickinson and Company))) was used by being diluted with the respective extraction solutions by 1/2560 times. In a case of the anti-H5 influenza immunochromatography kit, the test line density (ΔOD) is the density obtained when the H5 influenza HA protein was used by being diluted with the respective extraction solutions so as to yield 0.2 ng/mL.

The measurement results of the background density (OD) and the test line density (ΔOD) in the above Comparative Examples 1 and 2, Reference Example 1 and Examples 2, and 3 are shown in-Table 1. As shown in the results in Table 1, when BIGCHAP, CHAPS, and deoxycholic acid were used as the steroidal surfactant, the background density was decreased, and a sufficiently high test line density (ΔOD) was obtained. The test line density (ΔOD) of Examples 1 to 3 was at a level that could be judged by visual observation.

The size of the amplified silver particles was 6 µm to 8 µm.

**[Table 1]**

| | | Background density (OD) | Test line density (ΔOD) |
|---|---|---|---|
| Anti-influenza A immunochromatography kit | Comparative Example 1 | 0.823±0.00 | 0.002 |
| | Reference Example 1 | 0.482±0.03 | 0.080 |
| Anti-H5 influenza immunochromatography kit | Comparative Example 2 | 0.303±0.05 | 0.055 |
| | Example 2 | 0.268±0.02 | 0.092 |
| | Example 3 | 0.216±0.01 | 0.107 |

## Claims

1. An in vitro immunochromatography method comprising:
developing a complex of a substance to be tested and a labeling substance containing a metal modified with a first binding substance for the substance to be tested on an insoluble carrier in the presence of a surfactant having a steroid skeleton while the substance to be tested and the labeling substance form the complex; and
detecting the complex of the substance to be tested by capturing the substance to be tested and the labeling substance in a detection site on the insoluble carrier containing a second binding substance for the substance to be tested or a substance that can bind to the first binding substance for the substance to be tested and amplifying signals from the captured labeling substance,
wherein
a solution which contains the substance to be tested and the surfactant and in which the surfactant concentration is 0.1% by mass to 10% by mass is developed on the insoluble carrier,
the labeling substance containing a metal is a metallic colloid and
the surfactant is N,N-bis(3-D-gluconamidopropyl)cholamide (BIGCHAP) or 3-[(3-cholamidopropyl)dimethylammonio]propanesulfonate (CHAPS).

2. The in vitro immunochromatography method according to Claim 1,
wherein the insoluble carrier is a porous carrier.

3. The in vitro immunochromatography method according to Claim 1,
wherein the metallic colloid is a gold colloid.

4. The in vitro immunochromatography method according to any one of Claims 1 to 3,
wherein the first binding substance is an antibody, and/or the second binding substance is an antibody.

## Patentansprüche

1. In-vitro-Immunochromatographieverfahren umfassend:
Entwickeln eines Komplexes aus einer zu testenden Substanz und einer Metall enthaltende Markierungssubstanz, die mit einer ersten bindenden Substanz für die zu testende Substanz modifiziert ist, auf einem unlöslichen Träger in Gegenwart eines Tensids mit einem Steroidgrundgerüst, wobei die zu testende Substanz und die Markierungssubstanz den Komplex bilden, und
Detektieren des Komplexes aus der zu testenden Substanz durch Einfangen der zu testenden Substanz und der Markierungssubstanz an einer Detektionsstelle auf dem unlöslichen Träger, der eine zweite bindende Substanz für die zu testende Substanz oder eine Substanz enthält, die an die erste bindende Substanz für die zu testende Substanz binden kann, und Verstärken der Signale der eingefangenen Markierungssubstanz,
worin
eine Lösung, die die zu testende Substanz und das Tensid enthält und in der die Tensidkonzentration 0,1 Masse-% bis 10 Masse-% beträgt, auf dem unlöslichen Träger entwickelt wird,
die das Metall enthaltende Markierungssubstanz ein Metallkolloid ist und
das Tensid N,N-Bis(3-D-gluconamidopropyl)cholamid (BIGCHAP) oder 3-[(3-Cholamidopropyl)dimethylammonio]propansulfonat (CHAPS) ist.

2. In-vitro-Immunochromatographieverfahren gemäß Anspruch 1,
worin der unlösliche Träger ein poröser Träger ist.

3. In-vitro-Immunochromatographieverfahren gemäß Anspruch 1,
worin das Metallkolloid ein Goldkolloid ist.

4. In-vitro-Immunochromatographieverfahren gemäß irgendeinem der Ansprüche 1 bis 3,
worin die erste bindende Substanz ein Antikörper ist und/oder die zweite bindende Substanz ein Antikörper ist.

## Revendications

1. Procédé d'immunochromatographie in vitro comprenant :
le développement d'un complexe d'une substance à tester et d'une substance de marquage contenant un métal modifié avec une première substance de liaison pour la substance à tester sur un support insoluble en présence d'un tensioactif présentant un squelette de stéroïde alors que la substance à tester et la substance de marquage forment le complexe ; et
la détection du complexe de la substance à tester par capture de la substance à tester et de la substance de marquage dans un site de détection sur le support insoluble contenant une seconde substance de liaison pour la substance à tester ou une substance qui peut se lier à la première substance de liaison pour la substance à tester et l'amplification de signaux à partir de la substance de marquage capturée,
dans lequel
une solution qui contient la substance à tester et le tensioactif et dans laquelle la concentration en tensioactif est de 0,1 % en masse à 10 % en masse est développée sur le support insoluble,
la substance de marquage contenant un métal est un colloïde métallique et
le tensioactif est le N,N-bis(3-D-gluconamidopropyl)cholamide (BIGCHAP) ou le 3-[(3-cholamidopropyl)diméthylammonio]propane-sulfonate (CHAPS).

2. Procédé d'immunochromatographie in vitro selon la revendication 1,
dans lequel le support insoluble est un support poreux.

3. Procédé d'immunochromatographie in vitro selon la revendication 1,
dans lequel le colloïde métallique est un colloïde d'or.

4. Procédé d'immunochromatographie in vitro selon l'une quelconque des revendications 1 à 3,
dans lequel la première substance de liaison est un anticorps, et/ou la seconde substance de liaison est un anticorps.
